# EUROPEAN PATENT APPLICATION

(11) **EP 2 371 946 A1**
(43) Date of publication of application: **05.10.2011**
(21) Application number: 10158443.1
(22) Date of filing: 30.03.2010
(51) Int. Cl.: C12N 9/06

(54) **Improved methods and host cells for the production and use of D-amino acid oxidase (DAO).**

(71) Applicant: Rijksuniversiteit Groningen, 9712 CP Groningen (NL)
(72) Inventor: Van der Klei, Ida Johanna, 9751 NN Haren (NL); Veenhuis, Marten, 9751 NN Haren (NL); Klompmaker, Sandra Heidi, 9751 NN Haren (NL)
(74) Representative: Hatzmann, Martin

(57) **Abstract**

The invention relates to the field industrial application of enzymes and biocatalysis. More specifically, it relates to means and methods for improving processes which employ D-amino acid oxidase, in particular recombinantly produced D-amino acid oxidase. Provided is a method for the production of a catalytically active D-amino acid oxidase (DAO, EC 1.4.3.3) in a host cell, said host cell expressing a first nucleic acid sequence encoding DAO, comprising the step of transforming the host cell with a recombinant DNA molecule comprising at least a second nucleic acid sequence encoding a polypeptide comprising the transcarboxylation domain of pyruvate carboxylase (PYC, EC 6.4.1.1) or a functional homolog thereof.

## Description

The invention relates to the field industrial application of enzymes and biocatalysis. More specifically, it relates to means and methods for improving processes which employ D-amino acid oxidase, in particular recombinantly produced D-amino acid oxidase.

D-amino acid oxidase (DAO, EC 1.4.3.3) was first described in 1935 and since then has become a model enzyme for the dehydrogenase-oxidase family of flavoproteins (Krebs, 1935). It catalyzes the oxidative deamination of D-amino acids to the corresponding α-keto acid with a strict stereospecifity. A non-covalently bound FAD moiety is used as electron acceptor which reoxidizes via molecular oxygen to produce H₂O₂. DAO is predominantly observed in eukaryotes; however, one bacterial DAO was characterized recently (Geuke, *et al*., 2007).

DAO contains a peroxisomal targeting signal 1 (PTS1) in various eukaryote organisms and is imported into peroxisomes by the soluble receptor Pex5p (Yurimoto, *et al.,* 2000). Peroxisomes are ubiquitous and single membrane bound organelles. The physiological function, protein composition and abundance of peroxisomes strongly depend on the organism or cell type in which they occur and may alter with changes in environmental conditions (van der Klei & Veenhuis, 2006).

DAO has several possible industrially attractive applications. These include the analytical determination of D-amino acids, the separation of natural L- amino acid isomers from racemic mixtures and in the preparation of α-keto acids. The enzyme is used as a biocatalyst for industrial applications e.g. it catalyzes one of the key steps in the production of semisynthetic cephalosporins. DAO is especially important for the production of the cephalosporin antibiotics precursor 7-ACA (7-aminocephalosproanic acid). More than 50 different semi0synthetic cephalosprins are syntehsized from this antibiotic agent by modification of the side chain (reviewed by Pollegioni, *et al*., 2008).. Also, therapeutic applications have been proposed. For instance D-amino acid oxidase could be used in cancer treatment (for local formation of H₂O₂ to induce apoptosis in cancer cells).
A process for isolating the gene which codes for the DAO of *T. variabilis* and expressing it in *Escherichia coli* and in *T*. *variabilis* has been described (published Japanese Patent Application No. 71180/1988; European Patent Application No. 93202219.7, published as No. 0583817-A2). In addition, the gene which codes for the DAO of *F*. *solani* has also been cloned and expressed in *E. coli* and *Acremonium chrysogenum* (published Japanese Patent Application No. 2000181/1990; J. Biochem. (1990) 108:1063; Bio-Technology (1991) 9:188). In addition, it is known that the yeast *Rhodotorula gracilis* expresses DAO (Biotechnol. Appl. Biochem. (1992) 16:252). The advantages offered by the DAO *of R. gracilis* is that it has high catalytic efficiency (J. Biol. Chem. (1993) 268:13850) and a low dissociation constant of the FAD which is used as cofactor (Eur. J. Biochem. (1991) 197:513). The levels of DAO production by the wild strain *R. gracilis* are too low for an industrial process to be developed, however, US 6,187,574 discloses that the enzyme DAO of the yeast *R. gracilis* can be expressed in a prokaryotic microorganism such as *E. coli.* However, it is often a problem to produce this enzyme or D-amino acid oxidases from other species to high levels and in the enzymatically active form.

The aim of the present invention is therefore to improve the process for the production of (recombinant) DAO. In particular, it is aimed at providing a process with improved yield of catalytically active and stable DAO as compared to existing methods using a host cell.

Here, we report the presence of two DAO homologs in the yeast *P. pastoris.* One homolog has been identified as a bona fide D-amino acid oxidase. The enzyme shows activity towards D-alanine and has a pH optimum of 9.6. Our data suggest that the second homolog represents a D-aspartate oxidase. It was surprisingly found that in the yeast *Pichia pastoris,* the absence of pyruvate carboxylase (Pyclp) results in a strong decrease in DAO activity. Pyclp replenishes the citric acid cycle with oxaloacetate, but contains an additional ("moonlighting") function in the activation pathway of AO that is independent of its enzyme activity. Elaborating on this initial finding, it could further be demonstrated that (over)expression of at least part of the Pyc1 gene can improve the production of enzymatically active D amino acid oxidase enzyme by a host cell. Without wishing to be bound by theory, it is hypothesized that the transcarboxylation domain of pyruvate carboxylase plays a role in activation and/or stabilization of the DAO enzyme.

Accordingly, in one embodiment the invention provides a method for the production of a catalytically active D-amino acid oxidase (DAO, EC 1.4.3.3) in a host cell, said host cell expressing a first nucleic acid sequence encoding DAO, comprising the step of transforming the host cell with a recombinant DNA molecule comprising at least a second nucleic acid sequence encoding a polypeptide comprising the transcarboxylation domain of pyruvate carboxylase (PYC, EC 6.4.1.1) or a functional homolog thereof.

In another embodiment, the invention provides a non-naturally occurring (ie. recombinant) host cell expressing a first nucleic acid sequence encoding DAO, comprising the step of transforming the host cell with an expression vector comprising at least a second nucleic acid sequence encoding a polypeptide comprising the transcarboxylation domain of pyruvate carboxylase (PYC, EC 6.4.1.1) or a functional homolog thereof. Pyruvate carboxylase for use in the present invention can originate from any organism. In one embodiment, a eukaryotic sequence or fragment thereof is used. In another embodiment, a prokaryotic sequence or fragment thereof is used. For example, a bacterial or yeast pyruvate carboxylase. In a specific embodiment, *P*. *pastoris* Pyc1 is used..The term "transcarboxylation domain of pyruvate carboxylase" as used herein refers to the stretch of amino acid residues 479 to 1059 as found in the enzyme encoded by the *H. polymorpha* PYC1 gene [21].

A functional homolog is capable of enhancing the stability and/or activity of DAO. This pyruvate carboxylase does not have to be catalytically active to exert its stabilizing effect. In one embodiment, the polypeptide shows at least 70%, preferably at least 80%, more preferably at least 90%, most preferably at least 95% homology to the transcarboxylation domain sequence depicted in Figure....The skilled person will understand that one or more conservative amino acid substitutions can be made wherein one amino acid is replaced with another amino acid of similar chemical structure. Critical residues of Pyc1 are residues 479 to 1059, non-critical residues are residues 1 to 478 and 1060 to 1189.

Whereas the transcarboxylation domain of Pyc1 is thought to be most relevant for the advantageous effect on DAO, it may be preferred to express the full length pyruvate carboxylase since expression of a full length protein often results in the highest expression level. In one embodiment, the polypeptide shows at least 70%, preferably at least 80%, more preferably at least 90%, most preferably at least 95% homology to the Pyc1 amino acid sequence depicted in Figure....In a specific embodiment, the host cell is provided with a nucleic acid encoding a polypeptide showing at least 99% homology to the Pyc1 protein.

The invention can be practiced using any host cell capable of producing catalytically active DAO. Preferred host cells are those commonly used in industrial applications e.g. host cells which can be readily transformed and/or cultured at relatively high densities. In one embodiment it is a fungal cell, for instance a *Pichia pastoris, Hansenula polymorpha, Rhodotorula gracilis* or a *Penicillium chrysogenum* host cell. In another embodiment, the host cell is a bacterial host cell, preferably E. *coli, Bacillus brevis, Bacillus megaterium, Bacillus subtilis or Caulobacter crescentus.*

The invention is characterized by a host cell expressing DAO under conditions wherein at least the transcarboxylation domain of pyruvate carboxylase is (over)expressed. This is achieved by expressing a first nucleic acid sequence encoding DAO and a second nucleic acid sequence encoding PYC or a fragment thereof originate from the same organism. The first and/or second nucleic acid may but do not have to originate from the same organism. However, to allow for optimal protein-protein interaction and enzyme stabilizing/enhancing effect it may be desirable that DAO and PYC originate from the same organism. For instance, the combination of DAO and PYC derived from *P. pastoris* can be used.

The said first nucleic acid sequence encoding DAO and said second nucleic acid sequence encoding PYC or a fragment thereof can but do not have to originate from said host cell. In one embodiment, the invention employs a "homologous system" wherein DAO and PYC originate from the host cell wherein they are expressed. For instance, the host cell is *P. pastoris* expressing endogenous DAO and being transformed with a nucleic acid sequence encoding a polypeptide comprising the transcarboxylation domain of *P*. *pastoris* PYC, or a functional homolog thereof. As said, expression of full length PYC is preferred. Another preferred embodiment relates to a method or host cell, wherein said first nucleic acid sequence encoding DAO and said second nucleic acid sequence encoding PYC or a fragment thereof do not originate from said host cell. This is referred to as a 'heterologous system". Within a heterologous system, it may be advantageous that the first and second nucleic acid are derived from the same organism.

A further embodiment relates to a method for producing a compound of interest involving the oxidative deamination of a D-amino acid to the corresponding α-keto acid, comprising the steps of:
a) providing a host cell according to the invention,
b) culturing said host cell under conditions suitable for expressing catalytically active DAO; and
c) allowing said DAO to convert at least one D-amino acid to the corresponding
   α -keto-acid.

Step (a) typically comprises the transformation of the host cell with the necessary nucleic acid sequence(s) using standard molecular biological techniques. Any suitable expression vector may be used to provide the host cell with the transcarboxylation domain, and, depending on the host cell system used, the DAO gene. It may be practical to use an expression vector comprising a first nucleic acid sequence encoding DAO and at least a second nucleic acid sequence encoding a polypeptide comprising the transcarboxylation domain of pyruvate carboxylase or a functional homolog thereof. Preferably, said first and second nucleic acid sequence originate from the same organism.

In order to produce DAO, the host cell is grown in a medium containing a carbon source, a nitrogen source and mineral salts. The culturing conditions will depend on the host cell used. For instance, in case of an *E. coli* host cell a suitable temperature is between 18 and 30° C., and the pH must be kept between 5 and 9. Flasks of different volumes, from 50 ml to 1000 ml, with a quantity of medium between 10 and 50% of the volume of the flask, can be used for flask fermentation. One fermentation typically lasts for a period of between 12 and 90 hours. The skilled person will be able to determine optimal culturing conditions for any other type of (industrial) host cell. The cultivation of the recombinant micro-organisms can be improved if suitable conditions for maintaining the stability of the recombinant vectors are chosen- which is achieved by adding antibiotics to the culture medium, such as chloramphenicol, kanamycin or tetracycline, for which the recombinant vector containing the PYC gene presents a resistance marker. In addition to stability of production, this prevents the culture medium being contaminated with other unwanted micro-organisms and also eliminates the strains which, because they have lost the recombinant vector, do not display the enhanced DAO stability/activity. Typically, the DAO enzyme is isolated from the host cell and then used in an *in vitro* system to catalyze step the oxidative deamination of a D-amino acid to the corresponding α-keto acid. To that end, step (b) can be followed by harvesting DAO from the culture medium according to methods known in the art. For example, the DAO produced by the recombinant host cell can be extracted by separating the cells from the culture medium by centrifugation and subsequent disruption or permeabilization of the cells by a chemical, enzymatic or mechanical process. If an enzyme extract of greater purity is needed, the DAO can be purified by conventional precipitation, filtration or chromatographic, etc. techniques.

Accordingly, the invention also provides an enzyme composition comprising catalytically active DAO, said enzyme composition being obtainable from a host cell of the invention. The composition is characterized by a high content of catalytically active DAO. For example, it contains DAO activity in an amount of at least 50 U/mg of protein, preferably at least 70 U/mg of protein, more preferably at least 100 U/mg of protein. The (concentrated) enzyme extracts can also be immobilized by making them react with suitable inert solid supports, and in this way the immobilized DAO can be used cyclically. The invention also relates to the use of a (partially) purified polypeptide comprising at least the transcarboxylase domain of Pyclp or a functional fragment thereof to stabilize DAO. For instance, Pyclp can be added to an *in vitro* system wherein DAO is used as biocatalyst. Also within the scope of the invention is a method wherein the oxidative deamination of a D-amino acid is performed *in vivo,* i.e. within a host cell according to the invention.

As will be appreciated by a person skilled in the art, the invention finds its application in any process/system based on or involving the oxidative deamination of a D-amino acid to the corresponding α-keto acid. In one embodiment, it comprises determining the amount of D-amino acids in solution. In another embodiment, it is used in the separation of natural L-amino acids from a racemic mixture. In yet another embodiment, it is used for the preparation of α-keto acids.

A particularly attractive field for practicing the invention relates to the production of antibiotics. The DAO enzyme can be used for the preparation of 7-(5-oxoadipoamido) cephalosporanic acid. This acid is an intermediate for the preparation of 7-amino cephalosporanic acid, which in turn is a known intermediate for the preparation of a wide range of antibacterial agents of the cephalosporin family.
In one embodiment, the invention therefore provides a method for producing a cephalosporin, preferably 7-aminocephalosporanic acid (7-ACA) comprising the use of a host cell provided herein and/or an enzyme composition comprising DAO obtained from the host cell. For example, using the DAO obtained from a recombinant host cell of the invention which expresses the DAO gene of *R. gracilis* or another source, it is possible to produce GL-7ACA from cephalosporin C.

In one embodiment, there is provided an expression vector comprising a first nucleic acid sequence encoding DAO and at least a second nucleic acid sequence encoding a polypeptide comprising the transcarboxylation domain of pyruvate carboxylase or a functional homolog thereof. Also provided is a (prokaryotic) host cell comprising said vector.

Still further, the invention provides the use of an expression vector at least encoding a pyruvate carboxylase or a functional fragment thereof to enhance the stability and/or activity of D- amino acid oxidase in a host cell. It might be useful to reduce catalase expression in a host cell, because the spontaneous decarboxylation of 7-(5oxoadipoamido) cephalosporanic acid to glutary-7-amino cephalosporanic acid occurs only in the presence of H₂O₂.

### LEGENDS TO FIGURES

**Figure 1****.** Sequence alignment of D-amino acid oxidases from *Rhodotorula gracilis, Homo sapiens, Candida boidinii* and *Pichia pastoris.* Catalytically important residues are marked with an asterisk. Secondary structural elements from *R. gracilis* are depicted above the alignment (α, α-helix; β, β-strand).
**Figure 2****.** DAOa exhibits D-amino acid oxidase activity.
   Fig. 2A: spot assay of *P. pastoris* WT (PPY), *ΔDAOα* and *ΔDAOb* on solid media containing glucose and 0.6% D-alanine. Cells were grown for three days at 30°C. Fig. 2B: DAO specific enzyme activities, determined in crude extracts of glucose-D-alanine cultured cells. The activities in extracts of DAOa deficient cells are strongly decreased. Overexpression of DAOa under control of the inducible AOX promotor increases DAO enzyme activities to 2.3 U. mg protein⁻¹. The mean value of two independent experiments is shown.
**Figure 3****.** Daolp has an alkaline pH optimum.
   The specific activity of Daop1 was determined using extracts of PPY cells that overexpress Daolp. Daolp displays the highest activity between pH 9.5 and 10.0.
**Figure 4****.** DAOb exhibits D-aspartate oxidase activity.
   Specific D-aspartate oxidase (DASPO) activities were determined in crude extracts of glucose/D-alanine cultured cells (PPY). DAOb deficient strain shows no DASPO activity whereas in *ΔDAOα* cells the DASPO activities are comparable to those in WT cells.

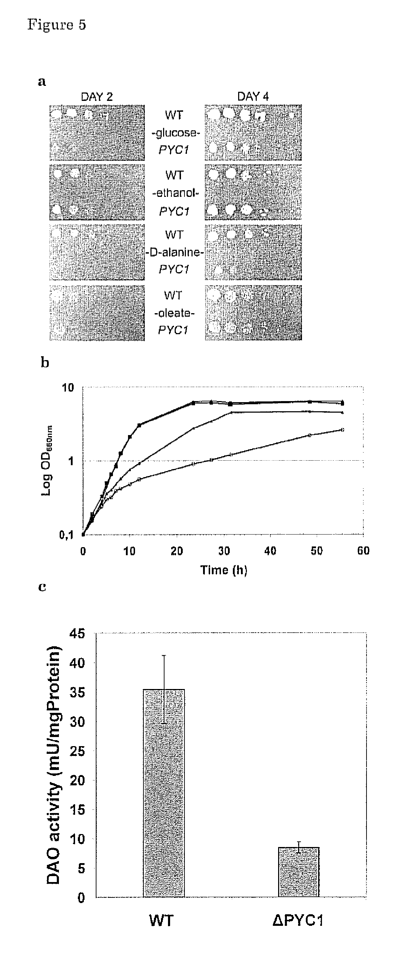

**Figure 5****.** Growth on D-alanine and DAO enzyme activities are decreased in *P*. *pastoris ΔPYC1* cells. Fig. 5A: spot assay of WT (MP36) and the *PYC1* cells derived from this strain on agar plates supplemented with respectively glucose/ NH₄(SO₄)₂, ethanol/ NH₄(SO₄)₂ , glucose/ D-alanine and oleate/ NH₄(SO₄)₂ as carbon and nitrogen sources. Cells were grown for two and four days at 30 _{°}C. *ΔPYC1* cells show a strong decrease in growth on glucose/ D-alanine but not at the other growth conditions. Fig. 5B: growth (expressed at absorption at 660 nm) of WT (◆) and *PYC1* **(■)** cells on ethanol/ L-aspartate. The WT and *ΔPYC1* cells grow similar on this nitrogen source. Cultivation of WT (▲) and *ΔPYC1* (o) cells on ethanol/ D-alanine shows that growth of the *ΔPYC1* strain is reduced. Fig. 5C: DAO activities were determined in crude extracts of WT and *ΔPYC1* cells grown on ethanol/ D-alanine. The activities in *ΔPYC1* cells were reduced to approximately 30% of the activities detected in WT cells. The mean value of two independent experiments is shown.

### EXPERIMENTAL SECTION

### EXAMPLE 1: Identification of a novel Pichia pastoris D-amino acid oxidase that is dependent on the function of pyruvate carboxylase for activation.

### MATERIALS AND METHODS

### Organisms and culture conditions

*P. pastoris* strains used in this study are listed in Table 1. All strains were cultured at 30°C. Media components were as follows: solid media contained 0.17% yeast nitrogen base, 2.2% agar, pH 6.0, with either 0.5% glucose or 0.3% ethanol in the presence of 0.5% ammonium sulphate. Oleate plates contained 0.1% oleic acid, 0.5% Tween 80, 0.1% yeast extract, 0.5% ammonium sulphate, 0.17% yeast nitrogen base and 2.2% agar, pH 6.0. D-alanine plates contained 0.6% D-alanine, 0.5% glucose, 0.17% yeast nitrogen base and 2.2% agar, pH 6.0. D-aspartate plates contained 0.6% D-aspartate, 0.5% glucose, 0.17% yeast nitrogen base and 2.2% agar, pH 6.0. Liquid mineral medium (van Dijken, *et al.,* 1976) was supplemented with 0.5% glucose and 0.25% ammonium sulphate or 0.5% methanol with either 0.25% ammonium sulphate or 0.25% D-alanine. Histidine, arginine and L-aspartate (60 mg/L) were added when needed.

**Table 1. P. pastoris strains used in example 1**

| Strain | Reference |
|---|---|
| MP36 *his3* | Menendez, 1998 |
| MP36 *his3, pyc1::HIS3* | Menendez, 1998 |
| PPY12 *his4, arg4* | Gould, 1992 |
| PPY12 *his4, arg4, RPPA05600Δ::zeocin* | This study |
| PPY12 *his4, arg4, RPPA06337Δ::hygromycin* | This study |
| PPY12 *his4, arg4, HIS4:: pPIC3.5K-DAO* | This study |

### Identification of PpDAO

A BLAST search of the *P. pastoris* genome database (Integrated Genomics, Chicago, USA) revealed two homologs, RPPA05600 and RPPA06337, of human D-amino acid oxidase (*HsDAO*)*.* Based upon homology to known genes and its function (see results), RPPA05600 was designated as *PpDAO.*

### Construction of deletion strains

The 0.2-Kbp 5' flanking region of RPPA05600 was amplified from PPY12 genomic DNA using primers SAK50 and SAK51 (Table 2). The 0.2-Kbp 3' flanking region of RPPA05600 was amplified by using oligo's SAK52 and SAK53. Both fragments were cloned into pBS-zeo, to obtain pBS-ΔRPPA05600. The plasmid pBS-zeo was constructed in the following way: the 1.1-Kbp zeocin cassette was amplified from pREMI-Z (van Dijk, *et al.,* 2001) using primers Zeo-XbaI-FW and Zeo-XhoI-RV and cloned into the *Xba*I/ *Xho*I fragment of pBluescript II KS+ (Stratagene, La Jolla, CA). The deletion cassette was amplified from pBS-ΔRPPA05600 with SAK50 and SAK53 and used to transform PPY12. Transformants were selected for their ability to grow in the presence of zeocin and checked by Southern blotting.
The 0.4-Kbp 5' flanking region of RPPA06337 was amplified from PPY12 genomic DNA with primers SAK54 and SAK55. The 0.2-Kbp 3' flanking region of RPPA06337 was amplified using oligos SAK56 and SAK57. Both fragments were cloned into pBS-del-PEX3-hph to obtain pBS-ΔRPPA06337. pBS-del-PEX3-hph was constructed in the following way: the *Sac*I site of pAG32 (Goldstein & McCusker, 1999) was removed by digestion, T4 DNA polymerase blunt ending and relegation, giving pAG32B. In the second step a 1.7-Kbp *Bgl*II/ *Eco*RV fragment of pAG32B was ligated to a 3.6-Kbp *Bgl*II/ *Eco*RV vector fragment of pRBG3. The deletion cassette was amplified from pBS-ΔRPPA06337 with SAK54 and SAK57 and used to transform PPY12. Transformants were selected for their ability to grow in the presence of hygromycin and checked by Southern blotting.

**Table 2. Oligonucleotides used in example 1**

| Primer | Sequence (5'→ 3') |
|---|---|
| SAK50 | GGGGAGCTCATCTCTAAATCCCCACTG |
| SAK51 | CCAAGCGGCCGCTTCTTGCCAGTGTTCAGAG |
| SAK52 | GGCTCGAGCTCCAAACAGATAGCTCATAC |
| SAK53 | GGTGAAGGTACCCTACAAGACGACAGTGCCG |
| SAK54 | GGATGTCGACCTTGCGGTGTCGCTACTTC |
| SAK55 | GCGAGATCTGTTGGGATGACCTCTCTC |
| SAK56 | GAGGATCCGCATCTTCTGCTGTCGTGC |
| SAK57 | CAGAGCTCGCTCGCTGAAATTTTGTGG |
| SAK94.1 | CGCGGATCCATGACTGATTCCAAATACGTTATCATTGG |
| SAK95.1 | GCAGGCGGCCGCTCATTTGGCAGCTTCAAAATAC |
| Zeo-XbaI-FW | CGCTCTAGAGTCATAGCTGTTTCCGATCCC |
| Zeo-XhoI-RV | CGCCTCGAGGCTCACATGTTGGTCTCCAGC |

### Ectopic expression

The full-length DAOa ORF was amplified from PPY12 genomic DNA using primers SAK94.1 and SAK95.1 and expressed from the AOX promoter in the vector pPIC3.5K (Invitrogen, Breda, The Netherlands). The resulting plasmid, designated pPIC3.5K-DAO, was linearized with *SalI* and transformed to PPY12 cells. Transformants were selected by histidine prototrophy and confirmed by Southern blotting.

### Molecular techniques

Oligonucleotides used in this study are presented in Table 2. All standard DNA-procedures were performed as described (Sambrook, *et al.,* 1989). Electro-transformation was performed according to Faber *et al.* (1994).

### Biochemical methods

D-amino acid oxidase and D-aspartate oxidase specific enzyme activities were measured spectrophotometrically at 30°C in crude cell extracts essentially as described previously (Sulter, *et al*., 1990). 5 µM FAD was added during the reaction. 50 mM D-alanine and 10 mM D-aspartate, respectively, were used as substrate. Protein concentrations were determined using the Bio-Rad assay kit and bovine serum albumin as a standard (Bio-Rad Gmbh, Munich, Germany). For determination of the pH optimum 100 mM sodium carbonate-bicarbonate buffer was utilized that buffers well between pH 8.5 and 11.5.

### RESULTS

### Identification of two D-amino acid oxidase homologues in P. pastoris

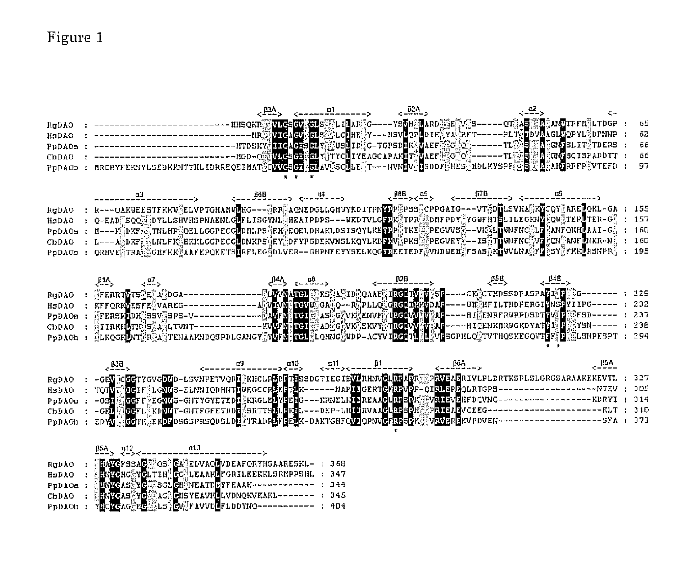

In order to search for D-amino acid oxidase homologues of *P. pastoris,* the gene coding for human D-amino acid oxidase (HsDAO) was used as query against the *P. pastoris* genome database. Two genes, RPPA05600 and RPPA06337, that code for two proteins of 344 and 404 amino acids, respectively, were identified. RPPA05600 was designated DAOa and RPPA06337 named DAOb. A database search reveals that DAOa shows the highest homology to *Candida boidinii* D-amino acid oxidase (66% identity). DAOb is 38% identical to CbDAO (Fig. 1). Both proteins contain the N-terminal GxGxxG nucleotide binding sequence that relates to the presence of a Rossman fold in the protein structure, a common structural feature of flavin-containing oxidoreductases. Neither DAOa nor DAOb contain a peroxisomal targeting signal type 1 or 2.

### DAOa exhibits D-amino acid oxidase activity

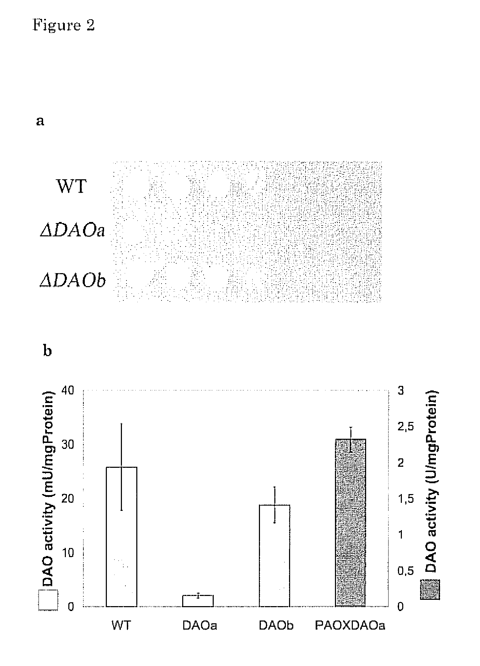

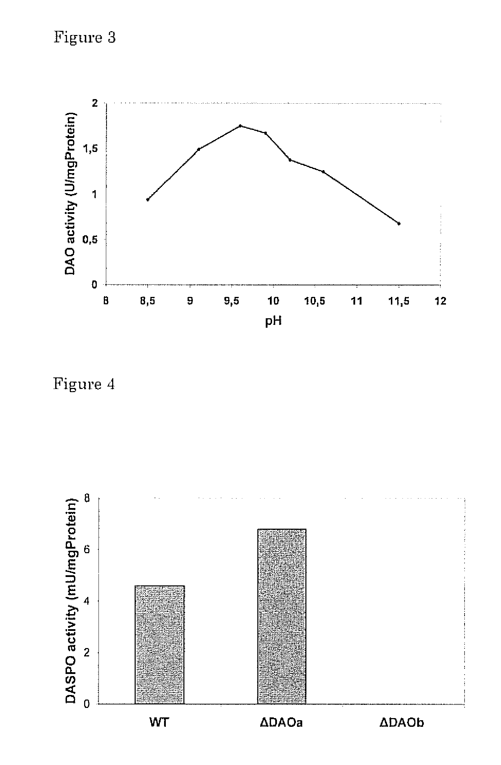

Deletion strains of DAOa and DAOb were constructed in the *P. pastoris* PPY12 background and analysed for defects in the utilization of D-alanine (Fig. 2), using a spot assay. Serial dilutions of cells were spotted on YND plates containing glucose in the presence of D-alanine as sole nitrogen source. The data presented in Fig. 2a shows that cells of a *ΔDAOa* strain display a strong growth defect, relative to *ΔDAOb* and the PPY12 host. This growth defect was accompagnied by a significant reduction in the DAO specific enzyme activity, detected in crude extracts prepared of D-alanine grown DAOa deficient cells (Fig. 2b). Identically grown cells of the *ΔDAOb* strain contained normal DAO activities comparable to those in WT controls. To confirm that the DAO activity is exhibited by the product of the DAOa gene, DAOa was overexpressed under control of the strong alcohol oxidase promoter. Cells of the transformants were grown on mineral media supplemented with methanol as sole carbon source to induce synthesis of DAOa. The DAO specific activities detected in crude cell extracts of these cells amounted to ~2.3 U.mg protein⁻¹, which is about 120 times higher than the endogenous WT activities. Based on these results, we designated the RPPA05600 gene DAO and its translation product PpDaolp.
The pH optimum of the enzyme was determined using crude extracts of Dao1p overexpressing cells (Fig. 3). The enzyme showed a remarkable high pH optimum of pH = 9.6. The pH optimum appeared to be rather broad and the enzyme functions well between pH 9.1 and 10.6.
Subsequently peptide antibodies were generated against Daolp. These antibodies recognized a specific protein band in Western blots, prepared from crude extracts of the Daolp overexpression strain that was absent in the Dao deletion strain (data not shown). However, in PPY12 WT cells also no protein band was detected. From this we conclude that Daolp is present at low levels in WT cells, consistent with their relative low specific activities that are below the limit of detection in the Western blot assays.

### DAOb exhibits D-aspartate oxidase activity

DAOa and DAOb also share approximately 35% sequence identity with human D-aspartate oxidase (DASPO). To determine whether one of the genes indeed displays D-aspartate oxidase activity, PPY12 and the DAOa and DAOb deletion strains were grown on glucose in the presence of D-aspartate as sole nitrogen source (Fig. 4). Enzyme measurements, using crude extracts of these cells, demonstrated that the WT host and *ΔDAOa* cells contained comparable amounts of aspartate oxidase activities whereas the enzyme activities were below the limit of detection in extracts of *ΔDAOb* cells.

### Growth on D-alanine and DAO enzyme activity are decreased in a ΔPYC1 strain

Previous reports showed that pyruvate carboxylase (Pyclp) assists the assembly of the peroxisomal flavo-enzyme alcohol oxidase (AO) in the yeasts *Hansenula polymorpha* and *P. pastoris* (Ozimek, *et al.,* 2003). This led us to investigate whether Pyclp also had a role in DAO assembly/activation. First we analyzed the growth properties of a *P. pastoris PYC* deletion strain (*PpPYC1*) on various carbon and nitrogen sources. The data depicted in Fig. 5a show that a strong reduction in growth was observed for the *PpPYC1* strain on glucose/ D-alanine. However, *PpPYC1* grew like the WT host on glucose/ (NH₄)₂SO₄,, ethanol/ (NH₄)₂SO₄ and oleate/ (NH₄)₂SO₄. Pyclp is an anaplerotic enzyme that replenishes the tricarboxylic acid cycle (TCA cycle) with oxaloacetate from pyruvate. This implies that *PYC1* cells of *P*. *pastoris* have to be supplemented with aspartate or glutamate to facilitate the cells to grow on glucose in liquid media (Stucka, *et al.,* 1991, Brewster, *et al.,* 1994). On ethanol however cells grow normally due to the induction of a bypass pathway, the glyoxylate cycle, which can complement for the withdrawal of intermediates for the TCA cycle. Therefore, additional growth experiments were performed in ethanol-containing liquid media supplemented with various nitrogen sources to assess whether the growth defect of a *PpPYC1* strain on D-alanine was specific for this nitrogen source (Fig. 5b). *PpPYC1* cells grew like WT on ethanol/ NH₄)₂SO₄ (data not shown) and ethanol/ L-aspartate. However, growth *of PpPYC1* cells on ethanol/ D-alanine is retarded, consistent with the growth data obtained on solid plates.
The observed defect *of PpPYC1* to grow on ethanol/ D-alanine was associated with a strong decrease (approximately 70%) in DAO specific enzyme activities relative to the activities detected in the WT host strain (Fig. 5c). This strongly suggests that in the absence of Pyclp normal assembly/ activation of PpDaolp is significantly disturbed.

### EXAMPLE 2: Expression of PYC1 enhances the recombinant production of DAO.

### Construction of Pichia pastoris PYC1 overexpression strains

The full-length PYC1 ORF is amplified from *P. pastoris* genomic DNA and expressed from the AOX promoter in the vector pPIC3.5K containing the *ARG4* marker (Invitrogen, Breda, The Netherlands). The resulting plasmid is linearized and transformed to *P. pastoris* wild type cells (PPY12 *his4 arg4*) or *P. pastoris* cells overproducing DAO (PPY12 *his4 arg4 HIS4::pPIC3.5K-DAO*). Correct integration is confirmed by Southern blotting.

### Analysis of DAO activity in PYC1 overexpression strains (homologous system)

Cells of *Pichia pastoris* WT, the DAO overproduction strains as well as both strains overproducing Pyc1 are induced by growth on methanol. DAO activities are measured in crude cell extracts.
A significant increase in DAO activity is observed upon co-overproduction of Pyc1 (see table 3)

**Table 3: DAO activities in the P. pastoris host cells**

| **Strain** | **Relative DAO enzyme activity** |
|---|---|
| WT | + |
| WT + Paox-PYC 1 | ++ |
| WT + P*_{AOX}*-DAO | ++++++ |
| WT + P*_{AOX}*-DAO + P*_{AOX}*-PYC1 | +++++++++++++ |

### Co-overexpression in Escherichia coli (heterologous system)

For production of PpDAO in a heterologous host, the gene is expressed in E. *coli.* A PCR-product encoding PpDAO with a C-terminal His-tag, is cloned into a pQE derived expression vector containing an amp resistance marker (Qiagen, Hilden, Germany). The resulting plasmid is designated pQE-DAO. In a similar way a pQE plasmid containing a kanamycin resistance marker is constructed containing a PCR product encoding PpPYC1with a C-terminal His6 tag (pQE-PYC1).

The plasmids are transformed to *E. coli* M15. Two strains are made. One containing only the pQE-DAO expression vector and a second strain containing both pQE-DAO and pQE-PYC1.

Transformants are grown as detailed in the *QIAexpressionist^{TM}.* Synthesis of the proteins is induced by the addition of 1 mM IPTG and incubation for 3 h at 30 °C. Synthesis of the proteins will be monitored by Western blotting using anti-His6 antibodies.

Co-production of PYC1 with DAO results in enhanced DAO activity (see table 4).

| **Strain** | **Relative DAO enzyme activity** |
|---|---|
| *E. coli* M15 | - |
| *E. coli* M15 + PpDAO | ++ |
| *E. coli* M15 + PpDAO + PpPYC1WT | ++++ |

### REFERENCES

[1] Brewster NK, Val DL, Walker ME & Wallace JC (1994) Arch Biochem Biophys 311: 62-71.
[2] Chumakov I, Blumenfeld M, Guerassimenko O, et al. (2002). Proc Natl Acad Sci U S A 99: 13675-13680.
[3] Decker BL & Wickner WT (2006) J Biol Chem 281: 14523-14528.
[4] Elgersma Y, Vos A, van den Berg M, van Roermund CW, van der Sluijs P, Distel B & Tabak HF (1996) J Biol Chem 271: 26375-26382.
[5] Evers ME, Titorenko V, Harder W, van der Klei IJ & Veenhuis M (1996) Yeast 12: 917-923.
[6] Faber KN, Haima P, Harder W, Veenhuis M & AB G (1994) Curr Genet 25: 305-310.
[7] Faber KN, Elgersma Y, Heyman JA, et al. (1998) Methods Mol Biol 103: 121-147.
[8] Gabler M, Hensel M & Fischer L (2000) Enzyme Microb Technol 27: 605-611.
[9] Gancedo C & Flores CL (2008). Microbiol Mol Biol Rev 72: 197-210.
[10] Geueke B, Weckbecker A & Hummel W (2007) Appl Microbiol Biotechnol 74: 1240-1247.
[11] Goldstein AL & McCusker JH (1999) Yeast 15: 1541-1553.
[12] Gunkel K, van Dijk R, Veenhuis M & van der Klei IJ (2004) Mol Biol Cell 15: 1347-1355.
[13] Harris CM, Pollegioni L & Ghisla S (2001) Eur J Biochem 268: 5504-5520.
[14] Islinger M, Li KW, Seitz J, Volkl A & Luers GH (2009) Traffic 10: 1711 - 1721.
[15] Klein AT, van den Berg M, Bottger G, Tabak HF & Distel B (2002) J Biol Chem 277: 25011-25019.
[16] Koller A, Spong AP, Luers GH & Subramani S (1999) Yeast 15: 1035-1044.
[17] Krebs HA (1935) Biochem J 29: 1620-1644.
[18] McCollum D, Monosov E & Subramani S (1993) J Cell Biol 121: 761-774.
[19] Neuberger G, Maurer-Stroh S, Eisenhaber B, Hartig A & Eisenhaber F (2003) J Mol Biol 328: 581-592.
[20] Odom AR, Stahlberg A, Wente SR & York JD (2000) Science 287: 2026-2029.
[21] Ozimek P, van Dijk R, Latchev K, Gancedo C, Wang DY, van der Klei IJ & Veenhuis M (2003). Mol Biol Cell 14: 786-797.
[22] Perotti ME, Pollegioni L & Pilone MS (1991) Eur J Cell Biol 55: 104-113.
[23] Perotti ME, Gavazzi E, Trussardo L, Malgaretti N & Curti B (1987) Histochem J 19: 157-169.
[24] Pollegioni L, Molla G, Sacchi S, Rosini E, Verga R & Pilone MS (2008) Appl Microbiol Biotechnol 78: 1-16.
[25] Saiardi A, Erdjument-Bromage H, Snowman AM, Tempst P & Snyder SH (1999). Curr Biol 9: 1323-1326.
[26] Sambrook J, Fritsch EF & Sambrook J (1989) Molecular cloning : a laboratory manual. Cold Spring Harbor Laboratory, Cold Spring Harbor, N.Y.
[27] Schlueter A, Fourcade S, Domenech-Estevez E, et al. (2007) Nucleic Acids Res 35: 815-822.
[28] Schrader T & Andreesen JR (1996) Appl Microbiol Biotechnol 45: 458-464.
[29] Stucka R, Dequin S, Salmon JM & Gancedo C (1991) Mol Gen Genet 229: 307-315.
[30] Sulter GJ, Waterham HR, Goodman JM & Veenhuis M (1990) Arch Microbiol 153: 485-489.
[31] Swinkels BW, Gould SJ & Subramani S (1992) FEBS Lett 305: 133-136.
[32] van der Klei IJ & Veenhuis M (2006) Biochim Biophys Acta 1763: 1364-1373.
[33] van Dijk R, Faber KN, Hammond AT, Glick BS, Veenhuis M & Kiel JA (2001) Mol Genet Genomics 266: 646-656.
[34] van Dijken JP, Otto R & Harder W (1976) Arch Microbiol 111: 137-144.
[35] Veenhuis M, van Dijken JP & Harder W (1976) Arch Microbiol 111: 123-135.
[36] Verrall L, Burnet PW, Betts JF & Harrison PJ (2009) Mol Psychiatry*.*
[37] Yurimoto H, Hasegawa T, Sakai Y & Kato N (2000) Yeast 16: 1217-1227.
[38] Yurimoto H, Hasegawa T, Sakai Y & Kato N (2001) Biosci Biotechnol Biochem 65: 627-633.

## Claims

1. A method for the production of a catalytically active D-amino acid oxidase (DAO, EC 1.4.3.3) in a host cell, said host cell expressing a first nucleic acid sequence encoding DAO, comprising the step of transforming the host cell with a recombinant DNA molecule comprising at least a second nucleic acid sequence encoding a polypeptide comprising the transcarboxylation domain of pyruvate carboxylase (PYC, EC 6.4.1.1) or a functional homolog thereof.

2. A non-naturally occurring host cell expressing a first nucleic acid sequence encoding DAO, comprising the step of providing said host cell with an expression vector comprising at least a second nucleic acid sequence encoding a polypeptide comprising the transcarboxylation domain of pyruvate carboxylase (PYC, EC 6.4.1.1) or a functional homolog thereof.

3. Method or host cell according to claim 1 or 2, wherein said expression vector encodes full length PYC.

4. Method or host cell according to any one of claim 1 to 3, wherein said host cell is a fungal cell, preferably selected from the group consisting of *Pichia pastoris, Hansenula polymorpha, Penicillium chrysogenum* and *Rhodotorula gracilis.*

5. Method or host cell according to any one of claim 1 to 4, wherein the host cell is a bacterial host cell, preferably *E*. *coli, Bacillus brevis, Bacillus megaterium, Bacillus subtilis or Caulobacter crescentus.*

6. Method or host cell according to any one of claim 1 to 5, wherein said first nucleic acid sequence encoding DAO and said second nucleic acid sequence encoding PYC or a fragment thereof originate from the same organism.

7. Method or host cell according to any one of claim 1 to 6, wherein said first nucleic acid sequence encoding DAO and/or said second nucleic acid sequence encoding PYC or a fragment thereof originate from said host cell.

8. Method or host cell according to claim 7, wherein said first nucleic acid sequence encoding DAO and said second nucleic acid sequence encoding PYC or a fragment thereof originate from said host cell.

9. Method for producing a compound of interest involving the oxidative deamination of a D-amino acid to the corresponding α-keto acid, comprising the steps of:
a) providing a host cell according to claim 2 and those depending thereon,
b) culturing said host cell under conditions suitable for expressing catalytically active DAO; and
c) allowing said DAO to convert at least one D-amino acid to the corresponding α-keto-acid.

10. Method according to claim 9, comprising determining the amount of D-amino acids, the separation of natural L-amino acids from a racemic mixture or the preparation of α-keto acids.

11. Method according to claim 9, comprising the production of a (semisynthetic) cephalosporin, preferably 7-aminocephalosporic acid (7-ACA).

12. Expression vector comprising a first nucleic acid sequence encoding DAO and at least a second nucleic acid sequence encoding a polypeptide comprising the transcarboxylation domain of pyruvate carboxylase or a functional homolog thereof.

13. Use of a polypeptide comprising the transcarboxylation domain of pyruvate carboxylase (PYC, EC 6.4.1.1) or a functional homolog thereof, or a nucleic acid sequence encoding said polypeptide, to enhance the stability and/or activity of DAO.
